# EUROPEAN PATENT SPECIFICATION

(11) **EP 1 725 298 B1**
(45) Date of publication and mention of the grant of the patent: **02.05.2007**
(21) Application number: 04818772.8
(22) Date of filing: 08.11.2004
(51) Int. Cl.: A61Q 5/12, A61K 8/41, A61K 8/20, A61K 8/34, A61K 8/36

(54) **HAIR CONDITIONING COMPOSITIONS**
HAARKONDITIONIERUNGSZUSAMMENSETZUNGEN
COMPOSITIONS D'APRES-SHAMPOOING

(30) Priority: 19.11.2003 FR 0313533
(43) Date of publication of application: 29.11.2006
(73) Proprietor: UNILEVER PLC, London, Greater London EC4P 4BQ (GB); UNILEVER N.V., 3013 AL Rotterdam (NL)
(72) Inventor: DAOU, Nancy, Lever Faberge France, 60880 Le Meux Cedex (FR); FRERE, Gaelle, Lever Faberge France, 60880 Le Meux Cedex (FR); SUN, Wei-Mei, Unilever Home & Personal Care USA, Chicago, IL 60008-4009 (US)
(74) Representative: Whaley, Christopher
(86) International application number: PCT/EP2004/012754
(87) International publication number: WO 2005/048961

(56) References cited:
- EP-A- 0 100 164
- EP-A- 0 956 850
- EP-A- 1 121 925

## Description

### Technical Field

The present invention is concerned with compositions for conditioning the hair which are typically applied to wet hair which has been shampooed and rinsed with water. The compositions are massaged into the hair followed by further rinsing with water before the hair is dried. Such compositions are known as rinse-off hair conditioners. In particular, the invention is concerned with improvements in the appearance, particularly the opacity of such compositions.

### Background and Prior art.

Rinse-off hair conditioners typically comprise as major components a cationic surfactant in combination with a fatty material, such as a fatty alcohol, in the form of an aqueous dispersion. The surfactant and alcohol are typically present in the composition at room temperature in the form of particles of lamellar liquid crystal (usually a so-called L beta lamellar liquid crystal) suspended in an aqueous solution.

Although the compositions thus formed do not transmit light well, they are not totally opaque. They would be better described as translucent, semi-opaque or opalescent. A corollary of this is that the compositions do not reflect or scatter light particularly well.

It has been found that many users of these products prefer the products to be opaque and to scatter light well. The resultant milky or creamy appearance conveys to the user that the composition has greater levels of active ingredients and hence will be more effective. Thus it is desirable to form hair conditioner compositions which are more opaque and which have a higher reflectance than prior art compositions.

One way to achieve this aim might be to disperse particles of a relatively high refractive index material throughout the composition. Such opacifiers are known to be used in hair treatment compositions (see for example the article by Norbert Boyxen in Olaj, Szappan, Kozmetika 50 (3) 2001). However there is a problem when such opacifiers are present in hair conditioner compositions. The opacifiers are non-functional ingredients, and may have poor biodegradability, particularly when high molecular weight polymer particles are used. The opacifiers may also deposit onto the hair and give the hair a dull, heavy or greasy feeling for some users.

Hence there is a need for an alternative technical route for making hair conditioning compositions appear opaque and milky or creamy.

Surprisingly, it has now been found that a particular combination of cationic surfactants and alkali metal halide salts can confer improved opacity and reflectance on hair conditioning compositions without the need for additional opacifiers.

### Summary of the invention

In one aspect, the invention provides a hair-conditioning composition comprising;
a) 60% by weight or more of water,
b) from 0.1 to 10% by weight of alkyl trimethylammonium salt wherein the alkyl group is selected from C₁₆ to C₂₂ saturated alkyl chains and mixtures thereof,
c) from 0.02 to 5% by weight of dialkoylethyl dimethylammonium salt wherein the alkyl chains are selected from C₁₆ to C₂₂ saturated or unsaturated alkyl chains and mixtures thereof,
d) from 0.5 to 10% by weight of a fatty material, comprising from 12 to 22 carbon atoms, selected from the group consisting of fatty alcohols, fatty acids, alkoxylated fatty alcohols and mixtures thereof, and
e) from 0.05 to 1% by weight of alkali metal halide,
wherein the weight ratio of the alkyl trimethylammonium salt to the dialkoylethyl dimethylammonium salt is from 15:1 to 2:1.

In another aspect, the invention provides a method of preparing an opacified hair conditioning composition by using a composition as described above.

A further aspect of the invention involves a method of treating the hair comprising the steps of;
i) applying to the hair a composition as described above,
ii) rinsing the hair with water and
iii) drying the hair.

### Detailed Description of the Invention.

Compositions according to the invention are aqueous compositions intended to be applied to the hair after shampooing and rinsing. They are massaged into wet hair and scalp, preferably followed by further rinsing with water prior to drying the hair. By aqueous composition, it is meant that the compositions of the invention comprise 60% by weight or more of water, preferably 70% or more, more preferably 80% or more.

### Cationic Surfactants

Compositions according to the invention comprise both an alkyl trimethylammonium salt and a dialkoylethyl dimethylammonium salt. These materials are both cationic surfactants.

The alkyl trimethylammonium salt is according to formula I

I R-N⁺(CH₃)₃X⁻

where R is a saturated alkyl chain with from 16 to 22 carbon atoms (i.e. C₁₆ to C₂₂). A mixture of such chains may be present in the alkyl trimethylammonium salt in compositions of the invention. X is preferably a halide or a methosulphate anion or mixtures thereof. Chloride is particularly preferred.

Particularly suitable alkyl trimethylammonium salts are cetyl trimethylammonium chloride (C₁₆) and behenyl trimethylammonium chloride (C₂₂).

The level of alkyl trimethylammonium salt in compositions of the invention should be from 0.1 to 10% by weight of the composition, preferably from 0.5 to 7%, more preferably from 1 to 5%.

The dialkoylethyl dimethylammonium salt needed for compositions of the invention is according to formula II:

II R₁COOCH₂CH₂-N⁺(CH₃)₂-CH₂CH₂OOCR₂X⁻

Wherein R₁ and R₂ are independently selected from C₁₅ to C₂₁ saturated or unsaturated alkyl chains. X is preferably a halide, a methosulphate anion or mixtures thereof. Chloride is particularly preferred.

In other words, the cation of the salt has the technical name N,N-dimethyl-2-[(1-oxoalkyl)oxy]-N-[2-[(2-oxoalkyl) oxy]ethyl] where the alkyl is from C₁₆ to C₂₂. These are referred to in the CTFA (8^{th} Edition 2000) as dialkoylethyl dimethylammonium salts

It is preferred if both R₁ and R₂ are the same alkyl group. A particularly preferred salt is dipalmitoylethyl dimethylammonium chloride, commercially available as Armocare VGH-70 (trade name) from Akzo GmbH.

The compositions according to the invention comprise from 0.02 to 5% by weight of the dialkoylethyl dimethylammonium salt, preferably from 0.1 to 3%, more preferably from 0.3 to 1.5%.

However, in addition to the constraints on levels of cationic surfactants in the compositions, the weight ratio of the alkyl trimethylammonium salt to the dialkoylethyl dimethylammonium salt must be from 15:1 to 2:1 to obtain compositions according to the invention. Preferably, the ratio is from 10:1 to 3:1, more preferably from 8:1 to 4:1.

### Fatty Material

Conditioner compositions of the invention comprise at least one fatty material. The combined use of fatty materials and cationic surfactants in the conditioning compositions is believed to lead to the formation of a structured lamellar or liquid crystal phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Suitable fatty materials comprise from 12 to 22 carbon atoms, preferably from 16 to 18 carbon atoms. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from 12 to 22 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty material in conditioners of the invention is suitably from 0.5 to 10, preferably from 1 to 6 percent by weight of the total composition. The weight ratio of cationic surfactant to fatty alcohol is suitably from 10:1 to 1:10, preferably from 4:1 to 1:8, optimally from 1:1 to 1:7.

### Alkali Metal Salt

An essential ingredient of the compositions according to the invention is an alkali metal halide. Mixtures of alkali metal halides may be used.

The alkali metal halide should be present as from 0.05% to 1% by weight of the composition. Too low a level leads to the improved reflectance of the compositions not being given, whereas too high a level can lead to separation of the compositions into separate aqueous and liquid crystalline phases, rather than remaining as a stable dispersion.

Preferably, the alkali metal halide is present as from 0.07 to 0.7% by weight of the composition, more preferably from 0.08 to 0.4%.

Preferred alkali metals are sodium or potassium and preferred halides are chlorides and bromides. Particularly preferred is potassium chloride.

### Process

A typical process for forming a hair conditioning composition comprising cationic surfactant and fatty material involves separately heating an aqueous dispersion or solution of the cationic surfactant, and the fatty material to a temperature above the melting point of the fatty material (typically 80 °C). The two components are then mixed together such that droplets of fatty material are dispersed as an emulsion in the surfactant solution. Upon cooling the emulsion, the surfactant and fatty material self-assemble into a lamellar liquid crystalline phase (L-beta phase) at some temperature lower than the melting point of the fatty acid but higher than 20°C.

Such a process is suitable for preparing compositions according to the invention. Preferably the alkyl trimethylammonium salt and the dialkoylethyl dimethylammonium salt are mixed in the hot aqueous dispersion prior to addition of the fatty material.

The alkali metal halide can be added at any stage of the process, but it is preferred if it is added after cooling the composition to less than 30°C.

### Conditioning Oil

A preferred component of the compositions according to the invention is a hydrophobic conditioning oil. In order for such an oil to exist in the preferred form as discrete droplets in the compositions according to the invention, it must be water-insoluble. By water-insoluble is meant that the solubility in water at 25°C is 0.01% by weight or less.

It is preferred if the conditioning oil is non-volatile, by which it is meant that the vapour pressure of the oil at 25°C is less than 10 Pa.

As used herein, the term "conditioning oil" includes any material, which is used to give a particular conditioning benefit to hair. For example, suitable materials are those which deliver one or more benefits relating to shine, softness, combability, wet-handling, anti-static properties, protection against damage, body, volume, stylability and manageability.

Suitable hydrophobic conditioning oils are selected from hydrocarbon oils, fatty esters, silicone oils and mixtures thereof.

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from 12 to 30 carbon atoms. Branched chain hydrocarbon oils can and typically may contain higher numbers of carbon atoms. Also suitable are polymeric hydrocarbons of alkenyl monomers, such as C₂-C₆ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 2000, preferably from about 200 to about 1000, more preferably from about 300 to about 600.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene.

A commercially available material of this type is L-14 polybutene from Amoco Chemical Co. (Chicago, Ill., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons ranging from C₁₆H₃₄ to C₂₁H₄₄. Suitable commercially available materials of this type include Sirius M85 and Sirius M125, all available from Silkolene.

Suitable fatty esters are characterised by having at least 10 carbon atoms, and include esters with hydrocarbyl chains derived from fatty acids or alcohols, e.g., monocarboxylic acid esters, polyhydric alcohol esters, and di- and tricarboxylic acid esters. The hydrocarbyl radicals of the fatty esters hereof can also include or have covalently bonded thereto other compatible functionalities, such as amides and alkoxy moieties, such as ethoxy or ether linkages.

Monocarboxylic acid esters include esters of alcohols and/or acids of the formula R'COOR in which R' and R independently denote alkyl or alkenyl radicals and the sum of carbon atoms in R' and R is at least 10, preferably at least 20.

Specific examples include, for example, alkyl and alkenyl esters of fatty acids having aliphatic chains with from 10 to 22 carbon atoms, and alkyl and/or alkenyl fatty alcohol carboxylic acid esters having an alkyl and/or alkenyl alcohol-derived aliphatic chain with 10 to 22 carbon atoms, benzoate esters of fatty alcohols having from 12 to 20 carbon atoms.

The monocarboxylic acid ester need not necessarily contain at least one chain with at least 10 carbon atoms, so long as the total number of aliphatic chain carbon atoms is at least 10. Examples include isopropyl isostearate, hexyl laurate, isohexyl laurate, isohexyl palmitate, isopropyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, and oleyl adipate. Di- and trialkyl and alkenyl esters of carboxylic acids can also be used. These include, for example, esters of C₄-C₈ dicarboxylic acids such as C₁-C₂₂ esters (preferably C₁-C₆) of succinic acid, glutaric acid, adipic acid, hexanoic acid, heptanoic acid, and octanoic acid. Examples include diisopropyl adipate, diisohexyl adipate, and diisopropyl sebacate. Other specific examples include isocetyl stearoyl stearate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol monostearate, ethoxylated propylene glycol monostearate, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters and mono-, di-and triglycerides.

Particularly preferred fatty esters are mono-, di- and triglycerides, more specifically the mono-, di-, and triesters of glycerol and long chain carboxylic acids such as C₁-C₂₂ carboxylic acids. A variety of these types of materials can be obtained from vegetable and animal fats and oils, such as coconut oil, castor oil, safflower oil, sunflower oil, cottonseed oil, corn oil, olive oil, cod liver oil, almond oil, avocado oil, palm oil, sesame oil, peanut oil, lanolin and soybean oil. Synthetic oils include triolein and tristearin glyceryl dilaurate.

Specific examples of preferred materials include cocoa butter, palm stearin, sunflower oil, soyabean oil and coconut oil.

The oil may be blended with other materials in the discrete droplets present in compositions according to the invention.

It is preferred that the d(0.5) volume-based median particle diameter of the hydrophobic conditioning oil droplets in the composition is less than 100 micrometres, more preferably less than 40 micrometres, even more preferably less than 10 micrometres and most preferably less than 6 micrometres.

Larger particle diameters lead to problems in stabilising the composition from separation of components. Practical difficulties in making emulsion droplets with a median diameter of 0.02 micrometres or less are known to those skilled in the art. Thus it is preferred if the volume-based median diameter d(0.5) is greater than 0.02 micrometres, more preferably greater than 0.03 micrometres, even more preferably greater than 0.1 micrometres. Preferred ranges of median diameter can be formed by combining any of the preferred minimum diameters with any of the preferred maximum diameters.

Volume-based median droplet diameter d(0.5) may be measured by means of a laser light scattering technique, for example using a 2600D Particle Sizer from Malvern Instruments.

The total amount of hydrophobic conditioning oil present in the composition is preferably from 0.1% to 10 % by weight of the total composition more preferably from 0.2% to 6%, most preferably 0.5% to 4 %.

### Silicone Conditioning oils

Preferred hydrophobic conditioning oils for use in compositions according to the invention are silicones.

Suitable silicones for use as conditioning oils include polydiorganosiloxanes, in particular polydimethylsiloxanes which have the CTFA designation dimethicone. Also suitable for use in compositions of the invention are polydimethyl siloxanes having hydroxyl end groups, which have the CTFA designation dimethiconol.

It is preferred if the silicone oil also comprises a functionalised silicone. Suitable functionalised silicones include, for example, amino-, carboxy-, betaine-, quaternary ammonium-, carbohydrate-, hydroxy- and alkoxy-substituted silicones. Preferably, the functionalised silicone contains multiple substitutions.

For the avoidance of doubt, as regards hydroxyl-substituted silicones, a polydimethylsiloxane merely having hydroxyl end groups (which have the CTFA designation dimethiconol) is not considered a functionalised silicone within the present invention. However, a polydimethylsiloxane having hydroxyl substitutions along the polymer chain is considered a functionalised silicone.

Preferred functionalised silicones are amino-functionalised silicones. Suitable amino functionalised silicones are described in EP 455,185 (Helene Curtis) and include trimethylsilylamodimethicone as depicted below, and are sufficiently water insoluble so as to be useful in compositions of the invention:

Si(CH₃)₃-O-[Si(CH₃)₂-O-]ₓ-[Si(CH₃)(R-NH-CH₂CH₂NH₂)-O-]_{y}-Si(CH₃)₃

wherein x + y is a number from about 50 to about 500, and the weight percent amine functionality is in the range of from about 0.03% to about 8% by weight of the molecule, and wherein R is an alkylene group having from 2 to 5 carbon atoms. Preferably, the number x + y is in the range of from about 100 to about 300, and the weight percent amine functionality is in the range of from about 0.03% to 8% by weight of the molecule.

As expressed here, the weight percent amine functionality is measured by titrating a sample of the amino-functionalised silicone against alcoholic hydrochloric acid to the bromocresol green end point. The weight percent amine is calculated using a molecular weight of 45 (corresponding to CH₃- CH₂-NH₂).

Suitably, the weight percent amine functionality measured and calculated in this way is in the range from 0.03% to 8%, preferably from 0.5% to 4%.

An example of a commercially available amino-functionalised silicone useful in the silicone component of the composition of the invention is DC-8566 available from Dow Corning (INCI name: dimethyl, methyl (aminoethylaminoisobutyl) siloxane). This has a weight percent amine functionality of about 1.4%.

By "amino functional silicone" is meant a silicone containing at least one primary, secondary or tertiary amine group, or a quaternary ammonium group. Examples of suitable amino functional silicones include: polysiloxanes having the CTFA designation "amodimethicone". Specific examples of amino functional silicones suitable for use in the invention are the aminosilicone oils DC-8220, DC-8166, DC-8466, and DC-8950-114 (all ex Dow Corning), and GE 1149-75, (ex General Electric Silicones). Suitable quaternary silicone polymers are described in EP-A-0 530 974. A preferred quaternary silicone polymer is K3474, ex Goldschmidt.

Another preferred functional silicone for use as a component in the hydrophobic conditioning oil is an alkoxy-substituted silicone. Such molecules are known as silicone copolyols and have one or more polyethyleneoxide or polypropyleneoxide groups bonded to the silicone polymer backbone, optionally through an alkyl linking group.

A non-limiting example of a type of silicone copolyol useful in compositions of the invention has a molecular structure according to the formula depicted below:

Si(CH₃)₃[O-Si(CH₃)(A)]ₚ-[O-Si(CH₃)(B)]_{q}-O-Si(CH₃)₃

In this formula, A is an alkylene chain with from 1 to 22 carbon atoms, preferably 4 to 18, more preferably 10 to 16. B is a group with the structure: -(R)-(EO)ᵣ(PO)ₛ-OH wherein R is a linking group, preferably an alkylene group with 1 to 3 carbon atoms. Preferably R is -(CH₂)₂-. The mean values of r and s are 5 or more, preferably 10 or more, more preferably 15 or more. It is preferred if the mean values of r and s are 100 or less. In the formula, the value of p is suitably 10 or more, preferably 20 or more, more preferably 50 or more and most preferably 100 or more. The value of q is suitably from 1 to 20 wherein the ratio p/q is preferably 10 or more, more preferably 20 or more. The value of p + q is a number from 11 to 500, preferably from 50 to 300.

Suitable silicone copolyols have an HLB of 10 or less, preferably 7 or less, more preferably 4 or less. A suitable silicone copolyol material is DC5200, known as Lauryl PEG/PPG - 18/18 methicone (INCI name), available from Dow Corning.

It is preferred to use a combination of functional and non-functional silicones as the hydrophobic silicone conditioning oil. Preferably the silicones are blended into common droplets prior to incorporation into compositions according to the invention.

The viscosity of the droplets hydrophobic silicone conditioning oil, measured in isolation from the rest of the composition (i.e. not the viscosity of any pre-formed emulsion, but of the hydrophobic conditioning oil itself) is typically from 350 to 200,000,000 mm²sec⁻¹ at 25°C. Preferably the viscosity is at least 5,000 mm²sec⁻¹ at 25 °C, more preferably at least 10,000 mm²sec⁻¹. Preferably the viscosity does not exceed 20,000,000 mm²sec⁻¹, more preferably 10,000,000 mm²sec⁻¹, most preferably 5,000,000 mm² sec⁻¹.

Suitable methods for measuring the kinematic viscosity of silicone oils are known to those skilled in the art, e.g. capillary viscometers. For high viscosity silicones, a constant stress rheometer can also be used to measure dynamic viscosity which is related to kinematic viscosity by the density of the silicone. The viscosity is measured at a low shear rate, less than 10 s⁻¹, such that the silicone exhibits Newtonian behaviour (i.e. viscosity independent of shear rate).

It is preferred if silicones are added to the compositions of the invention as pre-formed emulsions, more preferably as microemulsions.

### Further Ingredients

Compositions according to the invention may also incorporate other cosmetically suitable ingredients, preferably at a level of 2% by weight or less. Suitable ingredients include: viscosity modifiers, preservatives, colouring agents, polyols such as glycerine and polypropylene glycol, chelating agents such as EDTA, antioxidants, fragrances, antimicrobials, antidandruff agents, cationic conditioning polymers, styling ingredients, sunscreens, proteins and hydrolysed proteins. Volatile silicones such as cyclomethicones may also be used in compositions of the invention.

### Use

Compositions according to the invention are intended for use in the preparation of opacified hair conditioning compositions, particularly rinse-off hair conditioning compositions. It is preferred if the compositions are free of other opacifiers, in particular it is preferred if the compositions have less than 0.01% by weight of ethylene glycol distearate, water-insoluble styrene or acrylic polymers or copolymers or metal oxides.

The compositions are preferably used by applying them to the hair, followed by rinsing the hair with water followed by drying. It is more preferred if the compositions are applied and massaged into hair which is already wet, following shampooing and subsequent rinsing.

The invention will now be further demonstrated and illustrated with reference to the following examples.

### Examples

Compositions were prepared, as described above, using the compositions detailed in tables 1 and 2. All figures in the tables refer to the ingredients as 100% active by weight percent in the compositions.

Example 1 is according to the invention whereas examples A and B are comparative examples. A has no alkali metal halide and B has the same level of cationic surfactant as 1, but no dialkoylethyl dimethylammonium salt. Similarly, example 2 is according to the invention, but with higher levels of cationic surfactant and fatty material. Examples C and D are comparative examples to example 2. C has no alkali metal halide and D has the same level of cationic surfactant as 1, but no dialkoylethyl dimethylammonium salt.

**Table 1**

| **Formulation** | **1** | **A** | **B** |
|---|---|---|---|
| CTAC | 2.39 | 2.39 | 2.89 |
| DEQ | 0.5 | 0.5 | - |
| KCl | 0.3 | - | 0.3 |
| Natrosol 250 | 0.2 | 0.2 | 0.2 |
| Laurex CS | 3 | 3 | 3 |
| Disodium EDTA | 0.1 | 0.1 | 0.1 |
| Glydant | 0.4 | 0.4 | 0.4 |
| Water | to 100 | to 100 | to 100 |

CTAC is cetyl trimethylammonium chloride supplied commercially as 30% active. DEQ is dipalmitoylethyl dimethylammonium chloride supplied commercially as 76% active. Laurex CS is cetyl alcohol. Glydant is DMDM hydantoin preservative. Natrosol 250 is a hydroxyethylcellulose thickener.

**Table 2**

| **Formulation** | **2** | **C** | **D** |
|---|---|---|---|
| CTAC | 4.79 | 4.79 | 5.79 |
| DEQ | 1 | 1 | - |
| KCl | 0.1 | - | 0.1 |
| Natrosol 250 | 0.2 | 0.2 | 0.2 |
| Laurex CS | 6 | 6 | 6 |
| Disodium EDTA | 0.1 | 0.1 | 0.1 |
| Glydant | 0.4 | 0.4 | 0.4 |
| Water | to 100 | to 100 | to 100 |

The samples were measured using a colorimeter (Chroma meter CR-400/410). Table 3 shows the measurement results obtained using the standard La*b* notation.

| **Example** | **L** | **-a*** | **-b*** |
|---|---|---|---|
| 1 | 78 | 0.8 | 4 |
| A | 64 | 0.5 | 3 |
| B | 73 | 0.6 | 4 |

It is clear from the results that the example according to the invention has greater lightness and hence opacity and reflectivity than the comparative examples. This is also demonstrated by the Delta E* values. For 1 versus A the value is 14 and for 1 versus B 5. Both of these values mean that 1 is visually perceivable as whiter and a more saturated colour than both A and B.

## Claims

1. A hair-conditioning composition comprising
a) 60% by weight or more of water,
b) from 0.1 to 10% by weight of alkyl trimethylammonium salt wherein the alkyl group is selected from C₁₆ to C₂₂ saturated alkyl chains and mixtures thereof,
c) from 0.02 to 5% by weight of dialkoylethyl dimethylammonium salt wherein the alkyl chains are selected from C₁₆ to C₂₂ saturated or unsaturated alkyl chains and mixtures thereof,
d) from 0.5 to 10% by weight of a fatty material, comprising from 12 to 22 carbon atoms, selected from the group consisting of fatty alcohols, fatty acids, alkoxylated fatty alcohols and mixtures thereof, and
e) from 0.05 to 1% by weight of alkali metal halide,
wherein the weight ratio of the alkyl trimethylammonium salt to the dialkoylethyl dimethylammonium salt is from 15:1 to 2:1.

2. A composition according to claim 1 wherein the fatty material is a monohydric primary alcohol with an alkyl chain selected from C₁₆ to C₁₈ saturated chains and mixtures thereof.

3. A composition according to any preceding claim wherein the alkali metal halide is potassium chloride.

4. A composition according to any preceding claim wherein the alkyl trimethylammonium salt has an anion selected from the group consisting of halides, methosulphate and mixtures thereof.

5. A composition according to any preceding claim wherein the dialkoylethyl dimethylammonium salt has an anion selected from the group consisting of halides, methosulphate and mixtures thereof.

6. A composition according to any preceding claim wherein the alkyl trimethylammonium salt is cetyl trimethylammonium chloride.

7. A composition according to any preceding claim wherein the dialkoylethyl dimethylammonium salt is dipalmitoylethyl dimethylammonium chloride.

8. A composition according to any preceding claim wherein the weight ratio of the alkyl trimethylammonium salt to the dialkoylethyl dimethylammonium salt is from 8:1 to 4:1

9. A composition according to any preceding claim which further comprises a hydrophobic conditioning oil.

10. A composition according to claim 9 wherein the hydrophobic conditioning oil is a silicone oil.

11. A method of preparing an opacified hair conditioning composition by using a composition according to any preceding claim.

12. A method of treating the hair comprising the steps of;
i) applying to the hair a composition according to any of claims 1 to 10,
ii) rinsing the hair with water, and
iii) drying the hair.

## Patentansprüche

1. Haar-konditionierende Zusammensetzung, umfassend
a) 60 Gew.-% oder mehr Wasser,
b) 0,1 bis 10 Gew.-% Alkyltrimethylammoniumsalz, worin die Alkylgruppe aus gesättigten C₁₆ bis C₂₂-Alkylketten und Gemischen davon ausgewählt ist,
c) 0,02 bis 5 Gew.-% Dialkoylethyldimethylammoniumsalz, worin die Alkylketten aus gesättigten oder ungesättigen C₁₆ bis C₂₂-Alkylketten und Gemischen davon ausgewählt sind,
d) 0,5 bis 10 Gew.-% eines Fettmaterials, umfassend 12 bis 22 Kohlenstoffatome, ausgewählt aus der Gruppe, bestehend aus Fettalkoholen, Fettsäuren, alkoxylierten Fettalkoholen und Gemischen davon, und
e) 0,05 bis 1 Gew.-% Alkalimetallhalogenid,
worin das Gewichtsverhältnis von dem Alkyltrimethylammoniumsalz zu dem Dialkoylethyldimethylammoniumsalz 15 : 1 bis 2 : 1 ist.

2. Zusammensetzung nach Anspruch 1, worin das Fettmaterial ein einwertiger primärer Alkohol mit einer Alkylkette, ausgewählt aus gesättigten C₁₆ bis C₁₈-Ketten und Gemischen davon, ist.

3. Zusammensetzung nach einem vorangehenden Anspruch, worin das Alkalimetallhalogenid Kaliumchlorid ist.

4. Zusammensetzung nach einem vorangehenden Anspruch, worin das Alkyltrimethylammoniumsalz ein Anion aufweist, ausgewählt aus der Gruppe, bestehend aus Halogeniden, Methosulfat und Gemischen davon.

5. Zusammensetzung nach einem vorangehenden Anspruch, worin das Dialkoylethyldimethylammoniumsalz ein Anion aufweist, ausgewählt aus der Gruppe, bestehend aus Halogeniden, Methosulfat und Gemischen davon.

6. Zusammensetzung nach einem vorangehenden Anspruch, worin das Alkyltrimethylammoniumsalz Cetyltrimethylammoniumchlorid darstellt.

7. Zusammensetzung nach einem vorangehenden Anspruch, worin das Dialkoylethyldimethylammoniumsalz Dipalmitoylethyldimethylammoniumchlorid darstellt.

8. Zusammensetzung nach einem vorangehenden Anspruch, worin das Gewichtsverhältnis von dem Alkyltrimethylammoniumsalz zu dem Dialkoylethyldimethylammoniumsalz 8 : 1 bis 4 : 1 ist.

9. Zusammensetzung nach einem vorangehenden Anspruch, die weiterhin ein hydrophobes konditionierendes Öl darstellt.

10. Zusammensetzung nach Anspruch 9, worin das hydrophobe konditionierende Öl ein Siliconöl darstellt.

11. Verfahren zur Herstellung einer opazifierenden Haar-konditionierenden Zusammensetzung durch Verwenden einer Zusammensetzung nach einem vorangehenden Anspruch.

12. Verfahren zum Behandeln des Haars, umfassend die Schritte von;
i) Auftragen auf das Haar einer Zusammensetzung nach einem der Ansprüche 1 bis 10,
ii) Spülen des Haars mit Wasser und
iii) Trocknen des Haars.

## Revendications

1. Composition d'après-shampoing comprenant :
a) 60 % en poids, ou plus, d'eau,
b) de 0,1 à 10 % en poids de sel d' alkyl triméthyl ammonium dans lequel le groupe alkyle est choisi parmi les chaînes alkyles saturées de C₁₆ à C₂₂ et les mélanges de celles-ci,
c) de 0,02 à 5 % en poids de sel de dialkoyléthyl diméthyl ammonium, dans lequel les chaînes alkyles sont choisies parmi les chaînes alkyles saturées et insaturées de C₁₆ à C₂₂, et les mélanges de celles-ci,
d) de 0,5 à 10 % en poids d'une matière grasse, comprenant de 12 à 22 atomes de carbone, choisie dans le groupe constitué des alcools gras, des acides gras, des alcools gras alkoxylés et des mélanges de ceux-ci, et
e) de 0,05 à 1 % en poids d'halogénure de métal alcalin,
dans laquelle le rapport entre le sel d'alkyl triméthyl ammonium et le sel de dialkoyléthyl diméthyl ammonium est de 15 :1 à 2 :1

2. Composition selon la revendication 1, dans laquelle la matière grasse est un alcool primaire monohydrique avec une chaîne alkyle choisie parmi les chaînes saturées en C₁₆ à C₁₈ et les mélanges de celles-ci.

3. Composition selon l'une quelconque des revendications précédentes, dans laquelle l'halogénure de métal alcalin est du chlorure de potassium.

4. Composition selon l'une quelconque des revendications précédentes, dans laquelle le sel d'alkyl triméthyl ammonium a un anion choisi dans le groupe constitué des halogénures, du métosulfate et des mélanges de ceux-ci.

5. Composition selon l'une quelconque des revendications précédentes, dans laquelle le sel de dialkoyléthyl diméthyl ammonium a un anion choisi dans le groupe constitué des halogénures, du métosulfate et des mélanges de ceux-ci.

6. Composition selon l'une quelconque des revendications précédentes, dans laquelle le sel d'alkyltriméthyl ammonium est un chlorure de cétyl triméthyl ammonium.

7. Composition selon l'une quelconque des revendications précédentes, dans laquelle le sel de dialkoyléthyl diméthyl ammonium est du chlorure de dipalmitoyléthyl diméthyl ammonium.

8. Composition selon l'une quelconque des revendications précédentes, dans laquelle le rapport pondéral entre le sel d'alkyl triméthyl ammonium et le sel de dialkoyléthyl diméthyl ammonium est de 8 :1 à 4 : 1.

9. Composition selon l'une quelconque des revendications précédentes, comprenant en outre une huile conditionnante hydrophobe.

10. Composition selon la revendication 9, dans laquelle l'huile conditionnante hydrophobe est une huile de silicone.

11. Procédé de préparation d'une composition d'après-shampoing opacifiée utilisant une composition selon l'une quelconque des revendications précédentes.

12. Procédé de traitement des cheveux comprenant les étapes consistant à :
i) appliquer aux cheveux une composition selon l'une quelconque des revendications 1 à 10,
ii) rincer les cheveux avec de l'eau, et
iii) sécher les cheveux.
